# EUROPEAN PATENT SPECIFICATION

(11) **EP 4 210 571 B1**
(45) Date of publication and mention of the grant of the patent: **02.04.2025**
(21) Application number: 21732137.1
(22) Date of filing: 25.05.2021
(51) Int. Cl.: A61B 5/11

(54) **DEVICE FOR THE OBJECTIVE CHARACTERIZATION OF SYMPTOMS OF PARKINSON'S DISEASE**
VORRICHTUNG ZUR OBJEKTIVEN CHARAKTERISIERUNG VON SYMPTOMEN DER PARKINSON-KRANKHEIT
DISPOSITIF POUR LA CARACTÉRISATION OBJECTIVE DE SYMPTÔMES DE LA MALADIE DE PARKINSON

(30) Priority: 10.09.2020 IT 202000021382
(43) Date of publication of application: 19.07.2023
(73) Proprietor: Brain Innovations S.r.l., 00128 Roma (IT)
(72) Inventor: DI BIASE, Lazzaro, 00128 ROMA (IT)
(74) Representative: De Gregori, Antonella
(86) International application number: PCT/IT2021/050160
(87) International publication number: WO 2022/054112

(56) References cited:
- CN-A- 109 480 858
- CN-A- 111 553 899
- SHARMA VINOD ET AL: "SPARK: Personalized Parkinson Disease Interventions through Synergy between a Smartphone and a Smartwatch", 1 January 2019, SPRINGER, PAGE(S) 103 - 114, XP047537470
- QI OUNG ET AL: "Technologies for Assessment of Motor Disorders in Parkinson's Disease: A Review", SENSORS, vol. 15, no. 9, 31 August 2015 (2015-08-31), pages 21710 - 21745, XP055746250, DOI: 10.3390/s150921710

## Description

The present invention refers to a device for the objective characterization of the symptoms of Parkinson's disease.

The invention is part of the technical field of medicine, in particular neurology.

Parkinson's disease is the most frequent neurodegenerative disease after Alzheimer's disease. The diagnosis of Parkinson's disease is essentially clinical and currently based on the objective and anamnestic identification of the characteristic signs and symptoms of the disease and on the exclusion of any atypical symptoms.

There are specific diagnostic criteria, developed over the years, in order to standardize and systematize the diagnosis of Parkinson's disease.

These methods objectively characterize the symptoms of Parkinson's disease, but do not integrate movement, tremor, facial and vocal analysis data and, above all, do not provide real-time data to monitor the progress of the disease moment by moment.

Patients with Parkinson's disease can experience fluctuations in their motor status during the day going from an OFF phase, in which parkinsonian symptoms such as rigidity, tremor and bradykinesia emerge, to an ON phase in which these symptoms improve significantly. marked, and also to a phase characterized by involuntary movements called dyskinesias (DYS) CN109480858 A discloses a wearable intelligent system for detecting a bradykinesia symptom of a patient suffering from a Parkinson's disease in a quantified manner. The system comprises a wristband equipped with an inertial sensor.

CN111553899 A discloses a system to detect Parkinson's disease based on audios and videos.

"SPARK: Personalized Parkinson Disease Interventions through Synergy between a Smartphone and a Smartwatch" by S. Vinod et al., in: Marcus, A. (eds) Design, "User Experience, and Usability. User Experience Design for Everyday Life Applications and Services", 2014 Lecture Notes in Computer Science, vol 8519, Springer, discloses a framework that combines information regarding facial tremors, dysfunctional speech, limb dyskinesia, and gait abnormalities. A hidden Markov model allows predicting an ON and OFF motor state.

"Technologies for Assessment of Motor Disorders in Parkinson's Disease: A Review" by Q.W. Oung et al., in Sensors (Basel), vol 15(9):21710-45. doi: 10.3390/s150921710, 2015, discloses motor fluctuations cycle of Parkinson's disease, including OFF, ON and dyskinesia state.

Object of the present invention is providing a system for characterizing the motor state of the patient, as defined in independent claim 1.

The objective characterization of the symptoms of Parkinson's disease is taking place through the analysis of the motor state of patients with said Parkinson's disease, in which said motor state may be in one of the following phases:
- OFF phase, in which parkinsonian symptoms such as rigidity, tremor and bradykinesia emerge;
- ON phase, in which said symptoms improve markedly;
- DYS phase, in which involuntary movements defined as dyskinesias emerge.

The characterization uses voice analysis, facial analysis, tremor analysis and movement analysis of said patient as parameters for detecting said symptoms, an algorithm being provided for using said detection parameters to determine the motor state of said patient.

The system for the objective characterization of the symptoms of Parkinson's disease includes:
- an electronic bracelet, equipped with an accelerometer, a magnetometer and a triaxial gyroscope, used to collect continuous data on tremor and movement;
- a camera to capture facial expressions, to be activated at the request of the patient;
- a recorder to record the patient's voice to be activated at the patient's request;
- a processing device, equipped with suitable software which, using a special algorithm, integrates movement, tremor, facial and vocal data to identify the motor state of the patient with Parkinson's disease (ON, OFF, DYS);
- a display showing the patient's motor status (ON, OFF, DYS).

According to a preferred embodiment, said camera, said recorder, said processing device, said display and the relative software are integrated in a palmtop/smartphone, whereby the electronic bracelet sends the combined data of movement and tremor analysis directly to said palmtop / smartphone, said palmtop/smartphone being provided with a display able to visualize the motor state of the patient: ON, OFF, DYS.

Preferred embodiments and non-trivial variants of the present invention form the subject of the dependent claims.

The advantage deriving from the use of the invention consists in the fact that it guarantees continuous monitoring 24 hours a day, integrating kinematics data with facial and voice analysis data.

It will be immediately obvious that innumerable variations and modifications (for example relating to shape, dimensions, arrangements and parts with equivalent functionality) can be made to what is described, without departing from the scope of the invention, which is defined in the attached claims.

It is understood that all attached claims form an integral part of the present description.

The present invention will be better described by a preferred embodiment, given by way of nonlimiting example, with reference to the attached drawings, in which:
- Figure 1 shows a system for the objective characterization of the symptoms of Parkinson's disease, according to the invention;
- Figure 2 shows a variant of the system according to the invention.

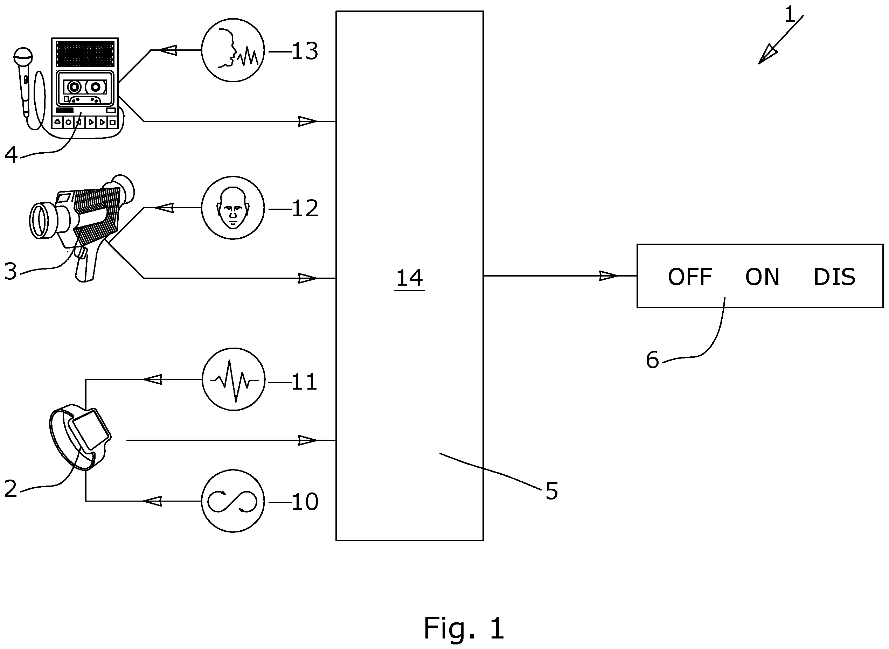

With reference to FIG. 1, the system (1) for the objective characterization of symptoms of Parkinson's disease includes:
- an electronic bracelet (2), equipped with an accelerometer, a magnetometer and a triaxial gyroscope, used to continuously collect data on tremor and movement;
- a camera (3) for recording facial expressions, to be activated at the request of the patient;
- a recorder (4) to record the voice to be activated at the request of the patient;
- a processing device (5), equipped with suitable software which, using a special algorithm, integrates movement, tremor, facial and vocal data to identify the motor state of the patient suffering from Parkinson's disease (ON, OFF, DYS);
- a display (6) showing the patient's motor status (ON, OFF, DYS).

This system is used for the objective characterization of the symptoms of Parkinson's disease.

The operation of the system (1) is divided into the following steps.

Step 1 - The electronic bracelet (2) continuously records the patient's movement (10) and tremor (11) data.

Step 2 - When the patient perceives a state of OFF or marked dyskinesias (DYS), he uses the camera (3) to capture facial expressions and the recorder (4) to record his voice.

Step 3 - The processing device (5) receives from the electronic bracelet (2), the camera (3) and the recorder (4) the combined data of movement (10), tremor (11), facial images (12) and item (13) and, through the motor state characterization algorithm, identifies the patient's motor state (14) and displays it on the display (6): ON, OFF, DYS.

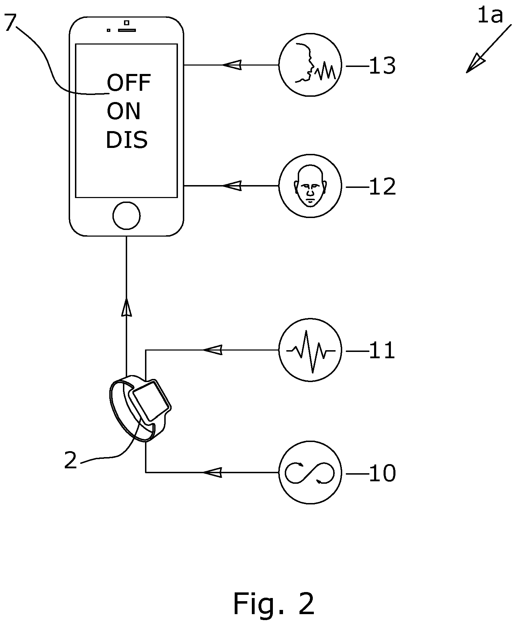

According to a preferred embodiment (1a) shown in FIG. 2, the camera (3), the recorder (4), the processing device (5), the display (6) and the related software are integrated in a PDA/smartphone (7), for which the electronic bracelet (2) sends the combined analysis data of movement (10) and tremor (11) directly to the PDA/smartphone (7), which carries out the analysis by applying the motor status characterization algorithm indicates on its display the patient's motor status: ON , OFF, DYS.

## Claims

1. System (1, 1a) for an objective characterization of symptoms of the Parkinson's disease, said characterization occurring through an analysis of the motor status of patients affected by said Parkinson's disease, wherein said system (1, 1a) comprises:
- an electronic bracelet (2), comprising an accelerometer, a magnetometer and a triaxial gyroscope, configured to collect data continuously about tremor and movement;
- a photo-camera (3) configured to shoot face expressions upon request of a patient;
- a recorder (4) configured to record the patient voice upon request of the patient;
- a processing device (5) configured to integrate data about movement, tremor, face and voice to identify the motor status of the patient affected by the Parkinson's disease, wherein said motor status can be in one of the following phases:
* OFF phase, wherein Parkinson's symptoms, such as rigidity, tremor and bradykinesia, emerge;
* ON phase, wherein said symptoms markedly improve;
* DYS phase, wherein involuntary movements emerge, called dyskinesias;
- a display (6) configured to display the motor status of the patient (ON, OFF, DYS).

2. System (1a) for the objective characterization of symptoms of the Parkinson's disease according to claim 1, **characterized in that** said photo-camera (3), said recorder (4), said processing device (5), said display (6) and the related software are integrated in a handheld computer/smartphone (7), so that the electronic bracelet (2) sends combined data about analysis of movement (10) and tremor (11) directly to said handheld computer/smartphone (7), said handheld computer/smartphone being equipped with a display adapted to display the motor status of the patient: ON, OFF, DYS.

## Patentansprüche

1. System (1, 1a) für eine objektive Charakterisierung von Symptomen der Parkinson-Krankheit, wobei die Charakterisierung durch eine Analyse des motorischen Status von Patienten erfolgt, die von der Parkinson-Krankheit betroffen sind, wobei das System (1, 1a) umfasst:
- ein elektronisches Armband mit einem Beschleunigungsmesser, einem Magnetomer und einem dreiachsigen Gyroskop, das so konfiguriert ist, dass es kontinuierlich Daten über Zittern und Bewegung erfasst;
- eine Fotokamera (3), die so konfiguriert ist, dass sie auf Wunsch des Patienten Gesichtsausdrücke aufnimmt;
- ein Aufzeichnungsgerät (4), das so konfiguriert ist, dass es die Patientenstimme auf Wunsch des Patienten aufzeichnet;
- eine Verarbeitungsvorrichtung (5), die so konfiguriert ist, dass sie Daten über Bewegung, Zittern, Gesicht und Stimme integriert, um den motorischen Status des von der Parkinson-Krankheit betroffenen Patienten zu identifizieren,
wobei der motorische Status in einer der folgenden Phasen sein kann:
* OFF-Phase, in der die Parkinson-Symptome wie Steifheit, Zittern und Bradykinesie auftreten;
* ON-Phase, in der sich die Symptome deutlich verbessern;
* DYS-Phase, in der unwillkürliche Bewegungen, so genannte Dyskinesien, auftreten;
- ein Display (6), das so konfiguriert ist, dass es den motorischen Status des Patienten anzeigt (ON, OFF, DYS)

2. System (1a) zur objektiven Charakterisierung von Symptomen der Parkinson-Krankheit nach Anspruch 1, **dadurch gekennzeichnet, dass** die Fotokamera (3), das Aufzeichnungsgerät (4), die Verarbeitungsvorrichtung (5), das Display (6) und die zugehörige Software in einen Handcomputer/Smartphone (7) integriert sind, so dass das elektronische Armband (2) kombinierte Daten über die Analyse der Bewegung (10) und des Zitterns (11) direkt an den Handcomputer/das Smartphone (7) sendet, wobei der Handcomputer/das Smartphone mit einem Display ausgestattet ist, das geeignet ist, den motorischen Status des Patienten anzuzeigen: ON, OFF, DYS.

## Revendications

1. Système (1, 1a) pour une caractérisation objective des symptômes de la maladie de Parkinson, ladite caractérisation se produisant par une analyse de l'état moteur des patients affectés par ladite maladie de Parkinson, dans lequel ledit système (1, 1a) comprend :
- un bracelet électronique comprenant un accéléromètre, un magnétomètre et un gyroscope triaxial, configuré pour collecter en continu des données sur les tremblements et les mouvements ;
- un appareil photo (3) configuré pour photographier les expressions faciales à la demande du patient ;
- un enregistreur ( 4) configuré pour enregistrer la voix du patient à la demande de ce dernier ;
- un dispositif de traitement (5) configuré pour intégrer les données relatives aux mouvements, aux tremblements, au visage et à la voix afin d'identifier l'état moteur du patient atteint de la maladie de Parkinson,
dans lequel ledit état moteur peut se trouver dans l'une des phases suivantes :
* la phase OFF, au cours de laquelle apparaissent les symptômes de la maladie de Parkinson, tels que la rigidité, les tremblements et la bradykinésie ;
* la phase ON, dans laquelle lesdits symptômes s'améliorent nettement ;
* la phase DYS, au cours de laquelle apparaissent des mouvements involontaires, appelés dyskinésies ;
- un écran (6) configuré pour afficher l'état moteur du patient (ON, OFF, DYS)

2. Système (1a) pour une caractérisation objective des symptômes de la maladie de Parkinson selon la revendication 1, **caractérisé en ce que** ledit appareil photo (3), ledit enregistreur (4), ledit dispositif de traitement (5), ledit écran (6) et le logiciel associé sont intégrés dans un ordinateur de poche/smartphone (7), de sorte que le bracelet électronique (2) envoie les données combinées relatives à l'analyse des mouvements (10) et des tremblements (11) directement à l'ordinateur de poche/smartphone (7), ledit ordinateur de poche/smartphone étant équipé d'un écran adapté à l'affichage de l'état moteur du patient : ON, OFF, DYS.
